# EUROPEAN PATENT APPLICATION

(11) **EP 4 375 357 A1**
(43) Date of publication of application: **29.05.2024**
(21) Application number: 22845839.4
(22) Date of filing: 14.07.2022
(51) Int. Cl.: C12M 1/00, C12M 3/00

(54) **METHOD FOR MANUFACTURING CELL CULTURE CONTAINER, AND CELL CULTURE CONTAINER**

(30) Priority: 21.07.2021 JP 2021120664
(71) Applicant: Toyo Seikan Group Holdings, Ltd., Shinagawa-ku Tokyo 141-8627 (JP)
(72) Inventor: KOSEKI, Osamu, Yokohama-shi, Kanagawa 240-0062 (JP); TANAKA, Satoshi, Yokohama-shi, Kanagawa 240-0062 (JP); TOTANI, Takahiko, Yokohama-shi, Kanagawa 240-0062 (JP); NISHIYAMA, Takaharu, Yokohama-shi, Kanagawa 240-0062 (JP)
(74) Representative: Hasegawa, Kan
(86) International application number: PCT/JP2022/027665
(87) International publication number: WO 2023/002905

(57) **Abstract**

A method for producing a cell culture vessel without a protrusion portion or a thin portion on a culture surface is provided. The method for producing the cell culture vessel having the culture surface of cells includes: using a primary mold (10) including a projecting portion (11) to form an uneven pattern structure (31) on a surface of a vessel base material (30) and a secondary mold (20) including a projecting portion (21, 22) to flatten a part of the surface of the vessel base material (30); pressing the primary mold (10) against the vessel base material (30) to form the uneven pattern structure (31) on the vessel base material (30); pressing the secondary mold (20) against a thin portion (33) formed on the vessel base material (30) to modify the thin portion (33) to a flattened portion (35); and configuring the surface of the vessel base material (30) including the uneven pattern structure (31) formed with at least a part of the modified flattened portion (35) as the culture surface to form the cell culture vessel.

## Description

### TECHNICAL FIELD

The present invention relates to a cell culture technique and especially relates to a method for producing a cell culture vessel having a fine uneven pattern structure on a culture surface.

### BACKGROUND ART

Recently, it has been demanded to efficiently culture the large amount of cells, tissues, and the like under an artificial environment in a medicinal product production and a field, such as gene therapy, regeneration medicine, and immunotherapy.

In such a situation, using a bag-shaped cell culture vessel made of thermoplastic resin, a large amount of cells are automatically cultured. It is proposed that, by forming a fine uneven pattern structure on a culture surface of the cell culture vessel, culture efficiency is improved.

A method for forming the fine uneven pattern structure on the culture surface generally includes a thermal transfer method that presses a flat plate-shaped or belt-shaped mold for forming the uneven pattern structure against a surface of a thermoplastic film and performs heating and pressurization, a method that brings a melted resin material in contact with a cooling roll surface for forming the uneven pattern structure and performs molding, and the like.

Although these methods are usable when a cell culture vessel having a culture surface with a usual size is produced, there was a problem that, when a cell culture vessel having a culture surface with a large size was produced, the methods were not easily applicable.

Patent Document 1: Japanese Patent No. 6075103
Patent Document 2: Japanese Patent No. 5102731

### DISCLOSURE OF THE INVENTION

### PROBLEMS TO BE SOLVED BY THE INVENTION

That is, to form the uneven pattern structure on the culture surface with the large size, a large-sized mold is required, and there was a problem that a production cost of the mold was enormous. Additionally, to use the large-sized mold, simply enlarging the mold fails to appropriately form the uneven pattern structure on the culture surface, and there was also a problem that a large-scale transfer apparatus for uniform pressurization of a large area was required.

On the other hand, a method that, without using a large-sized integrated mold, a plurality of small molds are arranged and used to dividedly process the culture surface and a method that a small mold is used to be divided into multiple times to dividedly process the culture surface are considered.

However, thus dividedly processing the culture surface generates large protrusions by flowing softened resin into a boundary of the molds and since the resin flows out at a peripheral edge portion of the mold, resins between the molds dividedly used multiple times and at a portion corresponding to the peripheral edge portion of the mold thin, causing a problem of facilitating cracks.

A risk of generating such cracks is a serious problem in production of the cell culture vessel, and the large protrusions on the culture surface caused a problem that sending a culture fluid was interfered. Furthermore, when protrusion portions are present on the culture surface, when a medium is discharged, a liquid thickness of the medium in the cell culture vessel cannot be smaller than a height of the protrusion portion, and therefore there was a problem that an amount of consumption of the medium in medium exchange was increased.

Especially, as long as a certain amount of a thickness is allowable, thickening a base material allows reducing cracks, and therefore the problem of the thinned resin does not cause a problem. However, for example, in a case of a bag-shaped cell culture vessel, since gas permeability to a vessel wall surface needs to be increased, there is a demand for thinning the thickness of the base material as much as possible. In view of this, in production of the cell culture vessel, the risk of generating cracks was a serious problem.

Here, Patent Document 1 discloses an endless belt-shaped metal mold that allows forming an uneven pattern structure on a large-sized culture surface. However, production of the belt-shaped molds is expensive, and there was a risk of tearing resin when a film on which the uneven pattern structure was formed was stripped from the mold.

Additionally, Patent Document 2 discloses a technique that arranges and disposes a plurality of molds to transfer a fine pattern formed on a surface of a mold. However, this technique relates to a nano-order display element for producing a largescreen liquid crystal display and is not thermal transfer but pattern formation to photocurable resin. Application of this mold to the thermal transfer flows the resin into a gap between the molds to generate protrusions. Accordingly, the problem when the culture surface is dividedly processed described above was not able to be solved.

Therefore, the inventors seriously studied to succeed the above-described problems and completed the present invention by using a primary mold including a projecting portion that forms an uneven pattern structure on a surface of a vessel base material and a secondary mold including a projecting portion for flattering a part of the surface of the vessel base material.

The present invention has been made in consideration of the circumstances, and an object of the present invention is to provide a method for producing a cell culture vessel and a cell culture vessel without a protrusion portion or a thin portion on a culture surface.

### SOLUTIONS TO THE PROBLEMS

To achieve the object, a method for producing a cell culture vessel of the present invention is a method for producing a cell culture vessel having a culture surface of cells that includes: using a primary mold including a projecting portion to form an uneven pattern structure on a surface of a vessel base material and a secondary mold including a projecting portion to flatten a part of the surface of the vessel base material; pressing the primary mold against the vessel base material to form the uneven pattern structure on the vessel base material; pressing the secondary mold against a thin portion formed on the vessel base material to modify the thin portion to a flattened portion; and configuring the surface of the vessel base material including the uneven pattern structure formed with at least a part of the modified flattened portion as the culture surface to form the cell culture vessel.

The method for producing the cell culture vessel of the present invention preferably includes: adjacently disposing a plurality of the primary molds along a boundary line and pressing the plurality of primary molds against the vessel base material to form the uneven pattern structure on the vessel base material; and pressing the secondary mold against a protruding portion formed at a portion corresponding to the boundary line of the plurality of primary molds and the thin portion in the vessel base material to modify the protruding portion and the thin portion to the flattened portions.

The method for producing the cell culture vessel of the present invention preferably includes: pressing the vessel base material multiple times using the one primary mold to form the uneven pattern structure on the vessel base material; and pressing the secondary mold against the thin portion formed at a portion corresponding to a peripheral edge portion of the primary mold in the vessel base material to modify the thin portion to the flattened portion.

The method for producing the cell culture vessel of the present invention preferably includes pressing the thin portion and a predetermined region inside the thin portion by the secondary mold.

The method for producing the cell culture vessel of the present invention preferably includes: bonding the vessel base material to a reinforcing material; and pressing the primary mold and the secondary mold against an opposite surface of a bonding surface of the reinforcing material in the vessel base material.

In the method for producing the cell culture vessel of the present invention, the primary mold and the secondary mold are preferably pressed to form the uneven pattern structure and the flattened portion on the vessel base material by thermal transfer or melt extrusion.

The method for producing the cell culture vessel of the present invention preferably includes: forming a plurality of approximately triangular prisms in parallel in a mountain range shape as the uneven pattern structure; or forming a plurality of depressed portions with or without a fine hole.

In the method for producing the cell culture vessel of the present invention, the cell culture vessel is preferably a bag-shaped vessel internally having the culture surface of the cells.

A cell culture vessel of the present invention is a bag-shaped cell culture vessel internally having a culture surface of cells that includes a plurality of regions, a flattened portion, and a flattened portion. On the plurality of regions, an uneven pattern structure is formed on the culture surface. The flattened portion is disposed between a plurality of regions on which the uneven pattern structure is formed. The flattened portion is at an entire peripheral edge of the culture surface.

In the cell culture vessel of the present invention, the uneven pattern structure is preferably a plurality of approximately triangular prisms disposed in parallel in a mountain range shape or a plurality of depressed portions with or without a fine hole.

### EFFECTS OF THE INVENTION

According to the present invention, the method for producing the cell culture vessel and the cell culture vessel without a protrusion portion or a thin portion on the culture surface can be provided.

### BRIEF DESCRIPTION OF THE DRAWINGS

Fig. 1 includes schematic diagrams illustrating a process of a method for producing a cell culture vessel according to an embodiment of the present invention.
Fig. 2 is a schematic diagram illustrating a state of a first process in the method for producing the cell culture vessel according to the embodiment of the present invention.
Fig. 3 includes schematic diagrams illustrating a state of performing the first process and a second process of the method for producing the cell culture vessel according to the embodiment of the present invention by thermal transfer.
Fig. 4 is a schematic diagram illustrating a state of disposing primary molds on a vessel base material and a secondary mold in the method for producing the cell culture vessel according to the embodiment of the present invention.
Fig. 5 includes schematic diagrams illustrating a modification of disposing the primary molds and a secondary mold in the method for producing the cell culture vessel according to the embodiment of the present invention.
Fig. 6 includes schematic diagrams illustrating the primary mold in the method for producing the cell culture vessel according to the embodiment of the present invention and a reference example thereof.
Fig. 7 includes schematic diagrams illustrating a reference example of a secondary mold in the method for producing the cell culture vessel according to the embodiment of the present invention.
Fig. 8 is a diagram showing results of a test 1 of the method for producing the cell culture vessel according to the embodiment of the present invention.
Fig. 9 is a diagram showing results of a test 2 of the method for producing the cell culture vessel according to the embodiment of the present invention.
Fig. 10 includes diagrams illustrating photographs of a protruding portion and a thin portion of a vessel base material obtained by Comparative Example 3 in a test 3 of the method for producing the cell culture vessel according to the embodiment of the present invention.
Fig. 11 includes diagrams illustrating photographs of a flattened portion modified from a protruding portion and a flattened portion modified from a thin portion obtained by Working Example 3 in the test 3 in the method for producing the cell culture vessel according to the embodiment of the present invention.
Fig. 12 is a diagram showing results of the test 3 of the method for producing the cell culture vessel according to the embodiment of the present invention.

### DESCRIPTION OF PREFERRED EMBODIMENTS

The following describes embodiments of a method for producing a cell culture vessel and a cell culture vessel of the present invention in detail with reference to the drawings. However, the present invention is not limited to specific content of the following embodiments and working examples described later.

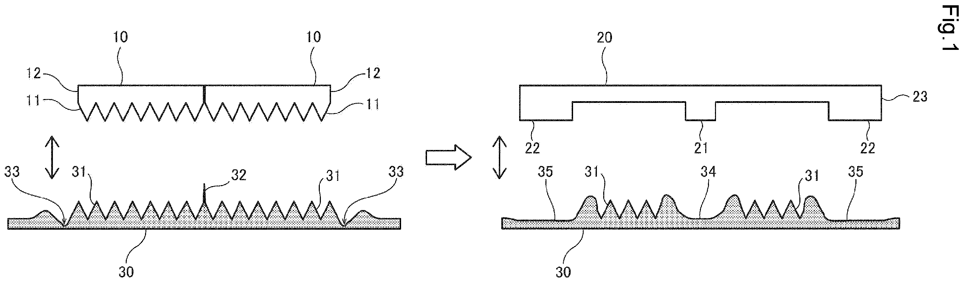

The method for producing the cell culture vessel of the embodiment is a method for producing a cell culture vessel having a culture surface of cells, and as illustrated in Fig. 1, uses primary molds 10 that include projecting portions 11 forming an uneven pattern structure 31 on a surface of a vessel base material 30 and a secondary mold 20 that includes projecting portions (21, 22) for flattering a part of the surface of the vessel base material 30.

The method for producing the cell culture vessel of the embodiment includes a first process that presses the primary molds 10 against the vessel base material 30 to form the uneven pattern structure 31 on the vessel base material 30.

Furthermore, the method for producing the cell culture vessel of the embodiment includes a second process that presses the secondary mold 20 against thin portions 33 formed on the vessel base material 30 by the first process to modify the thin portions 33 to flattened portions 35.

The method for producing the cell culture vessel of the embodiment forms the cell culture vessel with the surface of the vessel base material 30 including the uneven pattern structure 31 formed with at least a part of the modified flattened portions 35 as the culture surface.

In this Description and the claims, the flattened portion means a surface not having the uneven pattern structure but does not mean a complete flat and includes a case of being rounded.

The projecting portions 11 of the primary mold 10 only need to allow forming the uneven pattern structure on the vessel base material 30, and as illustrated in Fig. 1, may be one in which a plurality of triangular prisms are disposed in parallel in a mountain range shape (hereinafter referred to as a V pattern in some cases). In the diagram, the primary mold 10 is expressed as a cross-sectional view at a center in a depth direction viewed from a front side, and the projecting portion 11 is formed to have the triangular prism shape in the depth direction.

In the first process of the method for producing the cell culture vessel of the embodiment, a plurality of the primary molds 10 are preferably adjacently disposed along boundary lines and pressed against the vessel base material 30 to form the uneven pattern structure 31 on the vessel base material 30.

The method for producing the cell culture vessel of the embodiment preferably presses the secondary mold 20 against a protruding portion 32, which is formed at a portion corresponding to the boundary line of the plurality of primary molds 10 in the vessel base material 30, and the thin portions 33 to modify the protruding portion 32 and the thin portions 33 to the flattened portions (34, 35).

Furthermore, in the first process of the method for producing the cell culture vessel of the embodiment, the vessel base material 30 is preferably pressed multiple times using one primary mold 10 to form the uneven pattern structure 31 on the vessel base material 30.

In the method for producing the cell culture vessel of the embodiment, the secondary mold 20 is preferably pressed against the thin portions formed at the portions corresponding to the peripheral edge portions of the primary molds 10 (including the thin portion formed at the portion corresponding to the boundary line) in the vessel base material 30 to modify the thin portions to the flattened portions (34, 35).

The primary molds 10 and the secondary mold 20 are, for example, molds made of metal and silicon, in working examples described later, the primary mold 10 was manufactured using a silicon material and the secondary mold 20 was manufactured using an aluminum material. In this Description and the claims, a mold made of a material other than metal is also collectively referred to as a mold.

As the vessel base material 30, for example, a polyethylene sheet having a thickness of 0.1 mm can be used.

Specifically, as the material of the vessel base material 30, a resin film or the like can be preferably used and polyolefin-based resin or the like, such as polyethylene and polypropylene, can be used. Examples thereof can include polyethylene, a copolymer of ethylene and α-olefin, a copolymer of ethylene and vinyl acetate, and ionomer using ethylene and acrylic acid and/or methacrylic acid copolymers and metal ions. Additionally, polyolefin, styrene-based elastomer, polyester-based thermoplastic elastomer, and the like can be used. Furthermore, soft vinyl chloride resin, polybutadiene resin, ethylene-vinyl acetate copolymer, chlorinated polyethylene resin, polyurethane-based thermoplastic elastomer, polyester-based thermoplastic elastomer, silicone-based thermoplastic elastomer, styrene-based elastomer, for example, styrenebutadiene-styrene (SBS), styrene-isoprene-styrene (SIS), styrene-ethylene-butylene-styrene (SEBS), styrene-ethylene-propylene-styrene (SEPS), polyolefin resin, fluorinebased resin, and the like may be used.

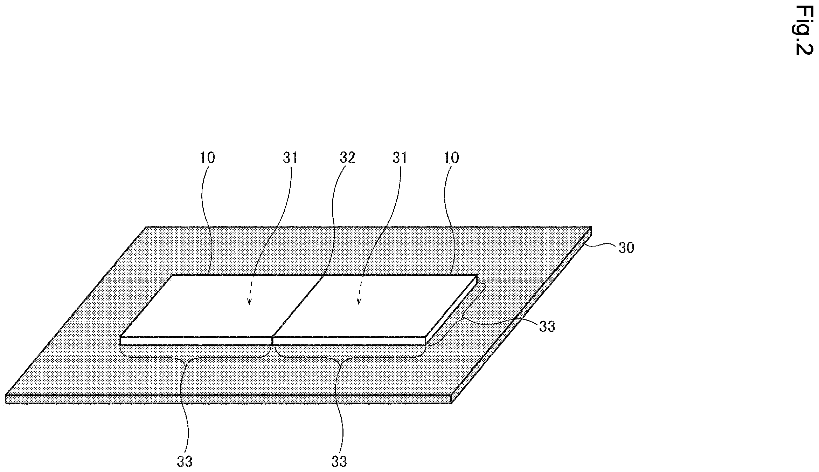

Fig. 2 illustrates an example of arrangement of the primary molds 10 and the vessel base material 30 in the first process.

In Fig. 2, the two primary molds 10 are disposed on the vessel base material 30, and when the primary molds 10 are pressed against the vessel base material 30, the uneven pattern structure 31 is formed on the surface of the vessel base material 30.

At this time, at the portion corresponding to the boundary line of the two primary molds 10 in the vessel base material 30, the protruding portion 32 is often formed as a result of the resin flowing into a slight gap between the primary molds 10. Additionally, at the peripheral edge portions of the primary molds 10 excluding the boundary line, as a result of the resin flowing out to the outside of the primary molds 10, the thin portions 33 having a thin thickness of the vessel base material 30 are often formed.

Producing the cell culture vessel using the vessel base material 30 in which the protruding portion 32 is formed causes a problem that solution sending of the medium is inhibited by the protruding portion 32. When the protruding portion 32 is present, a liquid thickness of the medium in the cell culture vessel cannot be smaller than a height of the protruding portion 32 when the medium is discharged, and this causes a problem that an amount of consumption of the medium in medium exchange increases.

When the cell culture vessel is produced using the vessel base material 30 in which the thin portions 33 are formed, a crack is likely to occur in the thin portion 33, and a problem of a liquid leakage of the medium occurs.

According to the method for producing the cell culture vessel of the embodiment, modifying the protruding portion 32 and the thin portion 33 to the flattened portions (a boundary flattened portion 34 and a peripheral edge flattened portion 35) using the secondary mold 20 allows producing the cell culture vessel without the protruding portion 32 or the thin portion 33, thereby ensuring solving the problems.

According to the method for producing the cell culture vessel of the embodiment, the flattened portions are formed in the cell culture vessel and a part of the uneven pattern structure 31 is squashed. However, this is a minor loss compared with the problems of the present invention and does not especially cause a problem in cell culture.

In the method for producing the cell culture vessel of the embodiment, the vessel base material 30 is preferably used by being bonded to a reinforcing material. That is, the vessel base material 30 is bonded to the reinforcing material, and the primary molds 10 and the secondary mold 20 are preferably pressed against an opposite surface of a bonding surface of the reinforcing material in the vessel base material 30.

By using the vessel base material 30 thus being bonded to the reinforcing material, the protruding portion 32 and the thin portions 33 formed on the vessel base material 30 by the primary molds 10 can be stably modified to the flattened portions by the secondary mold 20.

The reinforcing material preferably has a sticking layer, and the vessel base material 30 is preferably bonded and fixed to the sticking layer.

Here, simply stacking the vessel base material 30 on the reinforcing material provides small effect of stably modifying the protruding portion 32 and the thin portions 33 formed on the vessel base material 30 to the flattened portions by the secondary mold 20. On the other hand, by firmly sticking to the reinforcing material having the sticking layer harder than the vessel base material 30 (such as polyethylene), the effect can be sufficiently obtained.

That is, by sticking the reinforcing material to the vessel base material 30, thermal shrinkage when the vessel base material 30 is cooled in the thermal transfer in the first process and the second process is suppressed, and deformation, such as undulation of the vessel base material 30, can be suppressed. When the vessel base material 30 is stripped from the reinforcing material after terminating the thermal transfer, the presence of the sticking layer allows easily stripping the reinforcing material.

A material of the reinforcing material is not specifically limited, but a polyester-based material is suitable, and the use of polyethylene terephthalate (PET), which is harder than polyethylene, and easily available and inexpensive, is preferred. As a material of the sticking layer, for example, an acrylic-based adhesive material can be preferably used.

In the embodiment, as the material of the vessel base material 30, the use of the thermoplastic resin as described above is preferred. The uneven pattern structure 31 in the vessel base material 30 by pressing the primary molds 10 is preferably formed by thermal transfer or melt extrusion.

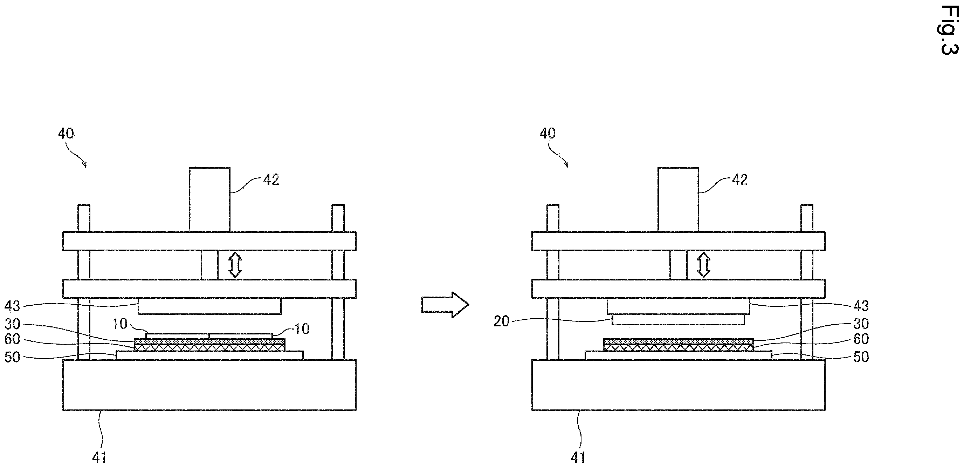

When the uneven pattern structure 31 in the vessel base material 30 is formed by thermal transfer, for example, a thermal transfer device 40 as illustrated in Fig. 3 can be used. That is, a buffer 50 (such as silicon rubber) is disposed on a baseplate 41, and the vessel base material 30 having a lower surface to which a reinforcing material 60 (such as polyethylene terephthalate) having a sticking layer is bonded is placed on the buffer 50. The plurality of primary molds 10 are disposed on an upper surface of the vessel base material 30, while a heating plate 43 is pressed against the primary molds 10 using a pressurization cylinder 42, the primary molds 10 are heated, and thus the first process can be performed.

Next, the primary molds 10 are removed from the upper surface of the vessel base material 30 to mount the secondary mold 20 on a lower surface of the heating plate 43. At this time, the secondary mold 20 is disposed such that a boundary projecting portion 21 in the secondary mold 20 is located above the protruding portion 32 formed on the vessel base material 30 and peripheral edge projecting portion 22 in the secondary mold 20 are located above the thin portions 33 formed on the vessel base material 30.

While the secondary mold 20 disposed on the heating plate 43 is pressed against the vessel base material 30 using the pressurization cylinder 42, the vessel base material 30 is heated, and thus the second process can be performed.

In the method for producing the cell culture vessel of the embodiment, a shape of the uneven pattern structure 31 formed on the vessel base material 30 is not specifically limited as long as having an uneven pattern. However, for example, the shape can be like the uneven pattern structure 31 as illustrated in the schematic diagram of Fig. 1 in which the plurality of triangular prisms are disposed in parallel in the mountain range shape (V pattern). In the diagram, the vessel base material 30 is expressed as a cross-sectional view at the center in the depth direction viewed from the front side, and the uneven pattern structure 31 is formed in the triangular prism shape in the depth direction.

The uneven pattern structure 31 formed on the vessel base material 30 may have a shape other than the above-described V pattern, and, for example, a plurality of triangular prisms may be disposed in parallel in a mountain range with intervals. Alternatively, a plurality of depressed portions (wells) may be formed, and the depressed portion may have a hemispherical shape, a conical shape, a polygonal pyramid shape, or the like, and the shape of the depressed portion is not specifically limited. Furthermore, the depressed portion may have or need not have a fine hole in a bottom.

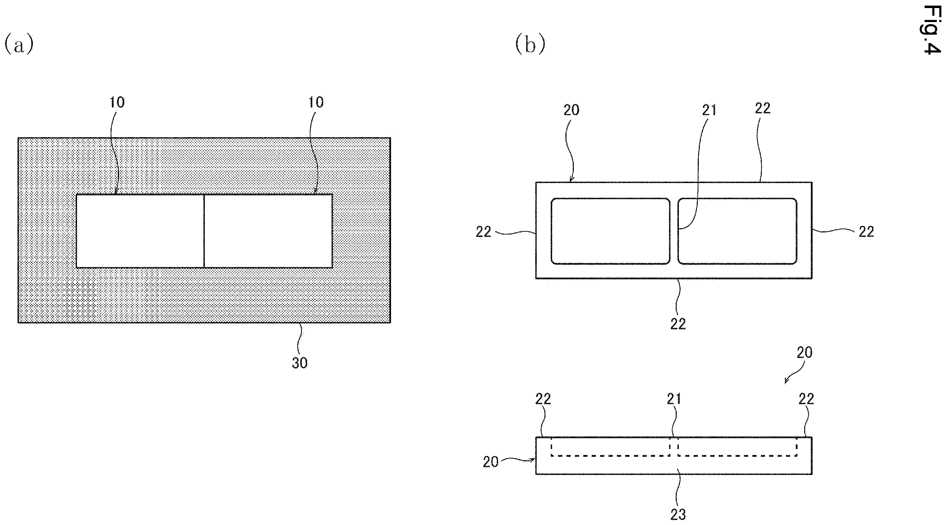

Next, with reference to Fig. 4, the primary molds 10 and the secondary mold 20 used in the method for producing the cell culture vessel of the embodiment will be further described in detail. Fig. 4(a) illustrates a state of the two primary molds 10 disposed on the vessel base material 30, Fig. 4(b) is a schematic diagram illustrating the structure of the secondary mold 20 used in this case, the upper side illustrates plan views and the lower side illustrates a front view.

As in Fig. 4(a), by disposing the two primary molds 10 on the vessel base material 30 and the primary molds 10 are pressed against the vessel base material 30, when the uneven pattern structure 31 is formed on the vessel base material 30, the protruding portion 32 is formed at the portion corresponding to the boundary line of the primary molds 10 in the vessel base material 30. The thin portions 33 are formed at the portions corresponding to the peripheral edge portions excluding the boundary line of the primary molds 10 in the vessel base material 30.

Therefore, in the embodiment, as in Fig. 4(b), using the secondary mold 20 including the boundary projecting portion 21 and the peripheral edge projecting portion 22, the vessel base material 30 on which the protruding portion 32 and the thin portions 33 are formed are pressed to ensure obtaining the vessel base material 30 without the protruding portion 32 or the thin portion 33.

That is, the secondary mold 20 includes the boundary projecting portion 21 to press the protruding portion 32 to modify it to the boundary flattened portion 34, the peripheral edge projecting portion 22 to press the thin portions 33 to modify them to the peripheral edge flattened portions 35, and a supporting portion 23 to support them. Heights of the boundary projecting portion 21 and the peripheral edge projecting portion 22 from a bottom surface of the supporting portion 23 are the same.

In Fig. 4(a), without using the two primary molds 10, pressing is repeatedly performed using one primary mold 10 to ensure forming the uneven pattern structure 31 on the vessel base material 30.

In this case, the thin portion 33 is formed at the portion corresponding to the boundary line of the primary mold 10 repeatedly used in the vessel base material 30, and the thin portions 33 are also formed at the portions corresponding to the peripheral edge portions excluding the boundary line of the primary molds 10 in the vessel base material 30.

In a case where the vessel base material 30 is pressed by thus using the primary mold 10 as well, the use of the secondary mold 20 including the boundary projecting portion 21 and the peripheral edge projecting portion 22 illustrated in Fig. 4(b) allows modifying the thin portions 33 to the flattened portions.

That is, the boundary projecting portion 21 allows modifying the thin portion 33 formed at the portion corresponding to the boundary line of the primary mold 10 to the boundary flattened portion 34, and the peripheral edge projecting portion 22 allows modifying the thin portions 33 formed at the peripheral edge portions of the primary mold 10 to the peripheral edge flattened portions 35.

Furthermore, in the method for producing the cell culture vessel of the embodiment, the peripheral edge projecting portion 22 is formed to have a wide width so as to ensure being pressed up to a predetermined region inside the thin portion 33. Thus, it is preferable that the thin portion 33 and the predetermined region inside the thin portion 33 are pressed by the secondary mold 20.

This is because when a bag-shaped vessel internally having a culture surface of cells as a cell culture vessel is formed, a peripheral area of the culture surface can be heat-sealed (thermally welded) so as to avoid a liquid leakage.

That is, in a case where the vessel base material 30 is heat-sealed to be a bag shape, for example, when the V pattern is formed as the uneven pattern structure 31, a V pattern formation surface needs to be an inner surface of the bag. In view of this, since the V pattern formation surface is located on the opposite surface of the vessel base material 30 with respect to a heat sealer, heat is not efficiently transmitted and it is difficult to completely weld.

Provisionally, when a temperature of the heat sealer is increased until the V pattern formation surface completely softens (welds), a V pattern portion can be completely welded (the V pattern disappears). However, since a part directly in contact with the heat sealer has a too high temperature to be burnt (carbonized), which is unpreferable.

Therefore, in the method for producing the cell culture vessel of the embodiment, to make the vessel base material 30 in the bag shape, the predetermined region where heat sealing is performed is preliminarily modified to the flattened portion by the secondary mold 20, thus ensuring preferable heat sealing.

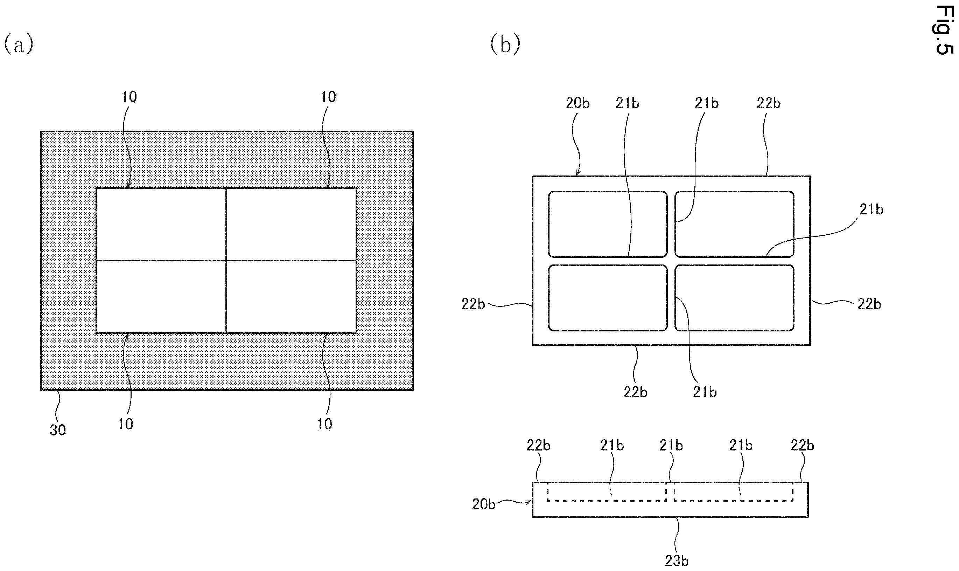

Next, with reference to Fig. 5, a modification of arrangement of the primary molds and the secondary mold in the method for producing the cell culture vessel of the embodiment will be described. Fig. 5(a) illustrates a state of disposing the four primary molds 10 on the vessel base material 30, Fig. 5(b) is a schematic diagram illustrating a structure of a secondary mold 20b used in this case, the upper side illustrates a plan view, and the lower side illustrates a front view.

As in Fig. 5(a), by disposing the four primary molds 10 on the vessel base material 30 and the primary molds 10 are pressed against the vessel base material 30, when the uneven pattern structure 31 is formed on the vessel base material 30, the protruding portion 32 is formed at the portion corresponding to the boundary line of the primary molds 10 in the vessel base material 30. The thin portions 33 are formed at the portions corresponding to the peripheral edge portions excluding the boundary line of the primary molds 10 in the vessel base material 30.

Therefore, in the embodiment, as in Fig. 5(b), using the secondary mold 20b including a boundary projecting portion 21b and the peripheral edge projecting portion 22b, the vessel base material 30 on which the protruding portion 32 and the thin portions 33 are formed are pressed to ensure obtaining the vessel base material 30 without the protruding portion 32 or the thin portion 33.

That is, the secondary mold 20b includes the boundary projecting portion 21b to press the protruding portion 32 to modify it to the boundary flattened portion 34, the peripheral edge projecting portion 22b to press the thin portions 33 to modify them to the peripheral edge flattened portions 35, and a supporting portion 23b to support them. Heights of the boundary projecting portion 21b and the peripheral edge projecting portion 22b from a bottom surface of the supporting portion 23b are the same.

In Fig. 5(a), without using the four primary molds 10, pressing is repeatedly performed using one or the two primary molds 10 to ensure forming the uneven pattern structure 31 on the vessel base material 30.

In this case, the thin portions 33 are formed at the portions corresponding to the boundary lines of the primary molds 10 repeatedly used in the vessel base material 30, and the thin portions 33 are also formed at the portions corresponding to the peripheral edge portions excluding the boundary lines of the primary molds 10 in the vessel base material 30.

In a case where the vessel base material 30 is pressed by thus using the primary molds 10 as well, the use of the secondary mold 20b including the boundary projecting portion 21b and the peripheral edge projecting portion 22b illustrated in Fig. 5(b) allows modifying the thin portions 33 to the flattened portions.

That is, the boundary projecting portion 21b allows modifying the thin portions 33 formed at the portions corresponding to the boundary lines of the primary molds 10 to the boundary flattened portions 34, and the peripheral edge projecting portion 22b allows modifying the thin portions 33 formed at the peripheral edge portions of the primary molds 10 to the peripheral edge flattened portions 35.

In the method for producing the cell culture vessel of the embodiment, as a method for forming the cell culture vessel with the surface of the vessel base material 30 including the flattened portions 34, 35 and the uneven pattern structure 31 as the culture surface, for example, the two vessel base materials 30 are prepared and the vessel base materials 30 are stacked such that the culture surface becomes the inside, thus ensuring heat-sealing the peripheral area.

Alternatively, one vessel base material 30 and one resin film on which a culture surface is not formed are prepared and they are stacked such that the culture surface becomes the inside, thus ensuring heat-sealing the peripheral area.

Besides, it is only necessary that the method for forming the cell culture vessel can form the surface of the vessel base material 30 including the flattened portions and the uneven pattern structure 31 described above as the culture surface, and specific means is not specifically limited.

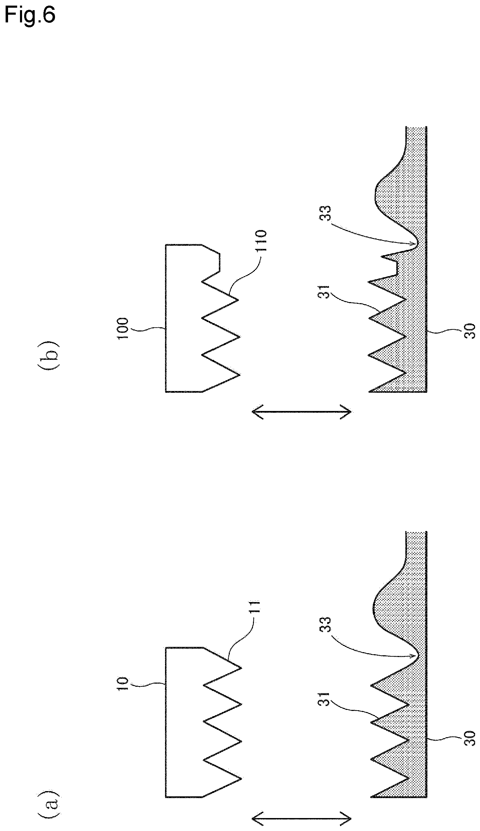

Here, with reference to Fig. 6, a primary mold in the method for producing the cell culture vessel of the embodiment and its reference example will be described.

Fig. 6(a) illustrates the primary mold 10 used in the method for producing the cell culture vessel of the embodiment and a state in which the vessel base material 30 is pressed by the primary mold 10 to form the thin portion 33 at the portion corresponding to the peripheral edge portion of the primary mold 10 in the vessel base material 30.

Prior to completing the present invention, as illustrated in Fig. 6(b), the inventors conducted an experiment on whether a thickness at a portion corresponding to the peripheral edge portion of the primary mold 10 in the vessel base material 30 increased and the thin portion 33 was not formed by cutting projecting portion 110 at peripheral edge portion of a primary mold 100 and pressing the primary mold 100 against the vessel base material 30.

However, it has been found that even when the primary mold 100 with the projecting portion 110 present at the peripheral edge portion is cut is used, as a result of the resin flowing out from the peripheral edge portion of the primary mold 10 when the primary mold 10 is pressed against the vessel base material 30, the thin portions 33 is formed after all.

Therefore, as a result of studies, the inventors solved the problem of forming the thin portions 33 at the portions corresponding to the peripheral edge portions of the primary molds 10 by using the primary molds 10 and the secondary mold 20 in combination and the thin portions 33 are modified to the flattened portions by the secondary mold 20.

In the case of using the primary mold 100 as well, the use of the secondary mold 20 allows eliminating the thin portion, and therefore the primary mold 100 can be used in the embodiment.

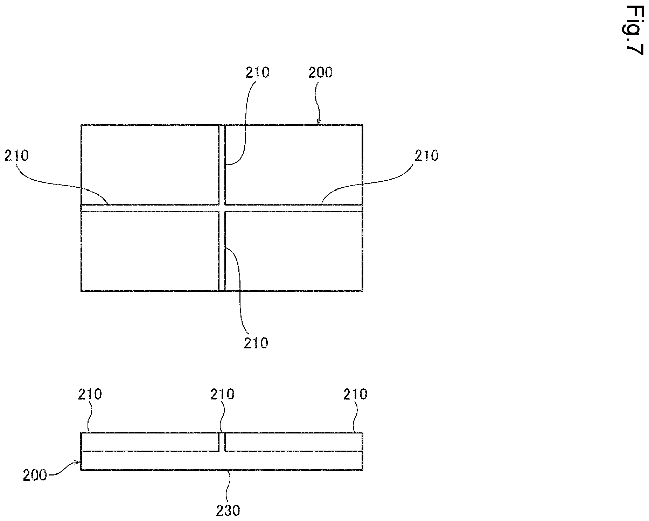

Next, with reference to Fig. 7, a reference example of a secondary mold in the method for producing the cell culture vessel of the embodiment will be described.

Fig. 7 is a schematic diagram illustrating a configuration of a secondary mold 200 that includes a boundary projecting portion 210 but does not include a peripheral edge projecting portion, the upper side illustrates a plan view, and the lower side illustrates a front view.

The use of the secondary mold 200 allowed squashing the protruding portion 32 in the vessel base material 30, but appropriately modifying the protruding portion 32 to the flattened portion was slightly difficult. That is, the use of the secondary mold 200 often deformed the whole vessel base material 30.

The use of the secondary mold 200 did not form the flattened portion in the peripheral area of the culture surface, and therefore when the vessel base material 30 was heat-sealed to be the bag shape, a gap of the uneven pattern structure 31 was not sufficiently welded to cause a liquid leakage in some cases.

Therefore, in the method for producing the cell culture vessel of the embodiment, the use of the secondary mold 20 including the peripheral edge projecting portion 22 together with the boundary projecting portion 21 allows stably modifying the protruding portion 32 to the flattened portion.

The cell culture vessel of the embodiment is obtained by the above-described method for producing the cell culture vessel, is a bag-shaped cell culture vessel internally having the culture surface of cells, and includes a plurality of regions on which the uneven pattern structure 31 is formed on the culture surface, the flattened portion 34 between the plurality of regions on which the uneven pattern structure 31 is formed, and the flattened portions 35 at the entire peripheral edge of the culture surface.

In the cell culture vessel of the embodiment, as long as the shape of the uneven pattern structure 31 is an uneven pattern, the shape is not specifically limited. However, for example, one in which the plurality of triangular prisms illustrated in the schematic diagram of Fig. 1 are disposed in parallel in the mountain range shape and one in which a plurality of triangular prisms are disposed in parallel in the mountain range shape with intervals may be used. A plurality of depressed portions (wells) maybe formed, and the shape may have or need not have a fine hole in a bottom of the depressed portion.

Cells cultured using the cell culture vessel of the embodiment are not especially limited, and may be floating cells, adherent cells, spheres (clumps of cells), or organoids generated by aggregating several kinds of cells.

As described above, according to the method for producing the cell culture vessel of the embodiment and the cell culture vessel, even when the plurality of primary molds are used or the primary mold is repeatedly used multiple times to form the uneven pattern structure on the vessel base material, the use of the secondary mold allows obtaining the vessel base material without the protruding portion or the thin portion. By using this, the cell culture vessel in which a crack is less likely to occur, solution sending of the medium is easily performed, and an amount of used medium can be reduced can be obtained.

### Working Example

The following will describe tests conducted to confirm the effects of the method for producing the cell culture vessel according to the embodiment of the present invention.

### [Test 1]

In the test, an experiment that simultaneously used the two primary molds to form the uneven pattern structure on the vessel base material, and then used the secondary mold to modify the protruding portion and the thin portion formed on the vessel base material to the flattened portions was conducted.

First, as the primary mold and the secondary mold used in the test, the following molds were manufactured.

As the primary mold, using a silicon (Si) material of 120 × 90 mm, a dicing process was performed with a blade having an angle to the horizontal was 75 degrees, and one having the plurality of triangular prisms like the primary mold 10 as in the schematic diagram of Fig. 1 was formed to be disposed in parallel in the mountain range shape in a longitudinal direction of the silicon material (hereinafter referred to as the V pattern in some cases) was manufactured. In the diagram, the primary molds 10 are expressed as a cross-sectional view at a center in a depth direction viewed from a front side, and the plurality of projecting portions 11 of the primary molds 10 are formed to be in a triangular prism shape in the depth direction.

The plurality of projecting portions in the primary molds were continuously formed without a gap from one another and the pitch (a width at a bottom side of a part of one projecting portion part) was 0.11 mm. A height of the projecting portion (a height from the bottom side to an apex of the projecting portion part) was 0.2 mm.

As the secondary mold, one that allowed muddying the protruding portion and the thin portions formed when the two primary molds were adjacently disposed in the longitudinal direction to the flattened portions was manufactured. Specifically, using an aluminum material of 180 × 120 mm, one including the boundary projecting portion 21 having a rectangular parallelepiped shape like the secondary mold 20 in the schematic diagram of Fig. 1 in a short side direction of the aluminum material was manufactured. At this time, the boundary projecting portion 21 was manufactured by cutting work. In the diagram, the secondary mold 20 is expressed as a cross-sectional view at a center in the depth direction viewed from a front view side and the boundary projecting portion 21 of the secondary mold 20 is formed in the rectangular parallelepiped shape in the depth direction.

The peripheral edge projecting portion 22 was formed on the peripheral edge portion of the secondary mold 20. As illustrated in Fig. 4, the peripheral edge projecting portion 22 was formed on the whole circumference of the secondary mold 20 having a width wider than the boundary projecting portion 21.

A width of the boundary projecting portion in the secondary mold was configured to be 2 mm and the width of the peripheral edge projecting portion 22 was configured to be 6 mm. Heights of the boundary projecting portion and the peripheral edge projecting portion were both configured to be 5 mm.

Here, although the width of the boundary projecting portion was configured to be 2 mm in the test, the size is not especially limited, and from an aspect of reducing the region of squashing the uneven pattern structure 31, the size is preferably narrow further. On the other hand, when the width of the boundary projecting portion is configured to be too narrow, positioning for modifying the protruding portion 32 to the flattened portion is difficult, and therefore the size was configured to be 2 mm in the test considering safety.

As the vessel base material, a linear low-density polyethylene film (manufactured by Toyo Seikan Group Holdings, Ltd.) having a thickness of 0.11 mm and 200 × 140 mm was used. The vessel base material was bonded to a reinforcing material made of polyethylene terephthalate using an acrylic-based adhesive material. At this time, the thickness of the reinforcing material was 0.06 mm, and a thickness of the sticking layer was 0.02 mm.

Next, on an opposite surface of a bonding surface of the reinforcing material in the vessel base material, as illustrated in Fig. 4(a), the two primary molds were adjacently disposed in the longitudinal direction and an uneven pattern structure was processed on a surface of the vessel base material using a thermal transfer apparatus (manufactured by Toyo Seikan Group Holdings, Ltd.). At this time, a temperature of the primary molds was set to be 130°C and pressurization was performed on the two primary molds at 4000 N for 20 seconds.

The surface of the vessel base material was set as a culture surface of the cell culture vessel, and a method for producing the cell culture vessel using only the primary molds was used as Comparative Example 1.

On the culture surface obtained by Comparative Example 1, a protruding portion was formed at a portion corresponding to a boundary line of the primary molds adjacent to one another. Thin portions were formed on peripheral edge portions excluding the boundary line of the primary molds. These protruding portion and thin portions were photographed from side surfaces using a microscope to measure a height of the protruding portion from a bottom surface of the vessel base material and a thickness of the vessel base material in the thin portion.

Next, the secondary mold was mounted on the thermal transfer apparatus and the secondary mold was pressed against the surface of the vessel base material on which the uneven pattern structure was processed. At this time, a temperature of the secondary mold was set to 130°C and pressurization was performed on the secondary mold at 4000 N for 20 seconds.

Accordingly, the secondary mold was pressed against the protruding portion formed at the portion corresponding to the boundary line of the primary molds adjacent to one another and the thin portions to modify the protruding portion and the thin portions to the flattened portions.

The surface of the vessel base material was set as a culture surface of the cell culture vessel, and a method for producing the cell culture vessel using the primary molds and the secondary mold was used as Working Example 1. The flattened portions were photographed from side surfaces using the microscope to measure a height of the flattened portion modified from the protruding portion from a bottom surface of the vessel base material and a thickness of the vessel base material in the flattened portion modified from the thin portion.

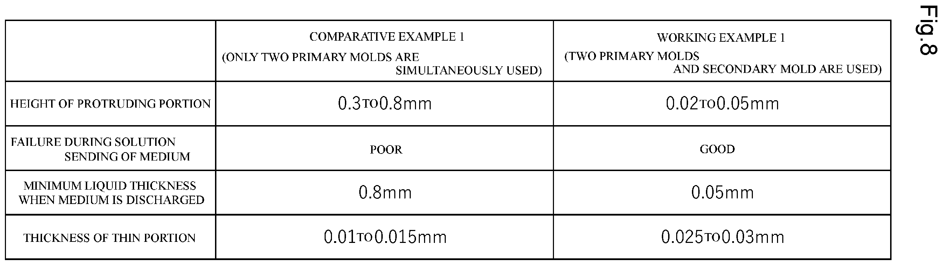

Fig. 8 shows results and effects of Comparative Example 1 and Working Example 1.

As shown in the diagram, the height of the protruding portions was from 0.3 to 0.8 mm, and a height at the highest portion of the protruding portion was 0.8 mm in Comparative Example 1. In contrast to this, the height of the flattened portions modified from the protruding portion was from 0.02 to 0.05 mm and the height of the highest portion of the flattened portion was 0.05 mm in Working Example 1.

Thus, it became apparent that while the protruding portion is present in the culture surface and therefore a failure possibly occurs during solution sending of the medium in Comparative Example 1, in Working Example 1, since the protruding portion is modified to the flattened portion, a risk of a failure during solution sending of the medium can be reduced.

It became apparent that since a minimum liquid thickness when the medium is discharged from the cell culture vessel was 0.8 mm in Comparative Example 1 but can be 0.05 mm in Working Example 1, the liquid thickness in the cell culture vessel can be decreased during medium exchange, and the amount of consumption of the medium can be reduced.

The thickness of the thin portions in Comparative Example 1 was from 0.01 to 0.015 mm, and a thickness of the thinnest portions of the thin portions was 0.01 mm. In contrast to this, the thickness of the flattened portions modified from the thin portions in Working Example 1 was from 0.025 to 0.03 mm, and the thickness of the thinnest portion of the flattened portion was 0.025 mm.

That is, the vessel base material obtained by Working Example 1 had no thin portion, and a crack was less likely to occur compared with the one obtained by Comparative Example 1.

### [Test 2]

In the test, an experiment that repeatedly used one primary mold twice to form the uneven pattern structure on the vessel base material, and then modified the thin portions formed on the vessel base material using the secondary mold to the flattened portions was conducted.

The same one as the test 1 was used as the primary mold.

A secondary mold that allowed modifying the protruding portion and the thin portions generated when the two primary molds were adjacently disposed in the short side direction to the flattened portions was manufactured. Specifically, using an aluminum material of 240 × 90 mm, it was manufactured by the method similar to the test 1.

A width of the boundary projecting portion in the secondary mold was configured to be 2 mm, and a width of the peripheral edge projecting portion 22 was configured to be 6 mm. Heights of the boundary projecting portion and the peripheral edge projecting portion were both 5 mm.

As the vessel base material, a linear low-density polyethylene film (manufactured by Toyo Seikan Group Holdings, Ltd.) having a thickness of 0.11 mm and 300 × 140 mm was used. The vessel base material was bonded to a reinforcing material made of polyethylene terephthalate using an acrylic-based adhesive material. At this time, the thickness of the reinforcing material was 0.06 mm, and a thickness of the sticking layer was 0.02 mm.

Next, on an opposite surface of a bonding surface of the reinforcing material in the vessel base material, one primary mold was disposed at one position where the two primary molds were able to be adjacently disposed in the short side direction and an uneven pattern structure was processed on a surface of the vessel base material using a thermal transfer apparatus (manufactured by Toyo Seikan Group Holdings, Ltd.).

At this time, a temperature of one primary mold was set to be 130°C and pressurization was performed on the one primary mold at 2000 N for 20 seconds.

Next, the primary mold was removed, one primary mold was disposed at the other position where the two primary molds were able to be adjacently disposed in the short side direction, and similarly, the second process of the uneven pattern structure was performed on the surface of the vessel base material.

The surface of the vessel base material was set as a culture surface of the cell culture vessel, and a method for producing the cell culture vessel using only the primary mold was used as Comparative Example 2.

On the culture surface obtained by Comparative Example 2, the thin portion was formed at the portion corresponding to the boundary line of the primary molds repeatedly used. The thin portion was also formed on the peripheral edge portion of the primary mold excluding the boundary line. The thin portions were photographed from side surfaces using the microscope to measure thicknesses of the vessel base material in the thin portions.

Next, the secondary mold was mounted on the thermal transfer apparatus and the secondary mold was pressed against the surface of the vessel base material on which the uneven pattern structure was processed. At this time, a temperature of the secondary mold was set to 130°C and pressurization was performed on the secondary mold at 4000 N for 20 seconds.

Accordingly, the secondary mold was pressed against the thin portions to modify the thin portions to the flattened portions.

The surface of the vessel base material was set as a culture surface of the cell culture vessel, and a method for producing the cell culture vessel using the primary mold and the secondary mold was used as Working Example 2. The flattened portions were photographed from side surfaces using the microscope to measure thicknesses of the vessel base material in the flattened portions modified from the thin portions.

Respective tensile strengths of the vessel base materials on which the uneven pattern structures were formed obtained by Comparative Example 2 and Working Example 2 were measured using a precision universal testing machine (Shimadzu Corporation, product number AG-IS). At this time, tension was performed in a direction parallel to the V pattern (the short side direction of the primary mold) formed on the vessel base material.

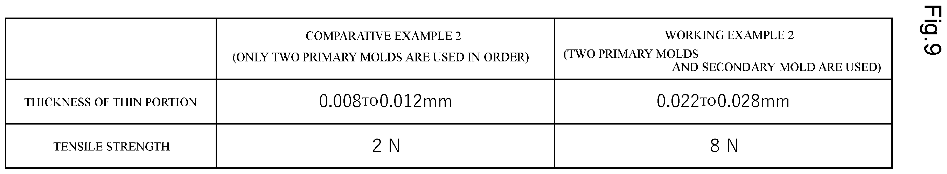

Fig. 9 shows results and effects of Comparative Example 2 and Working Example 2.

As shown in the diagram, the thickness of the thin portions was from 0.008 to 0.012 mm, and a thickness at the thinnest portion of the thin portions was 0.008 mm in Comparative Example 2. In contrast to this, the height of the flattened portions modified from the thin portions was from 0.022 to 0.028 mm and the thickness of the thinnest portion of the flattened portion was 0.022 mm in Working Example 2.

While the tensile strength of the vessel base material obtained by Comparative Example 2 was 2 N, the tensile strength of the vessel base material obtained by Working Example 2 was 8 N.

Accordingly, in the vessel base material obtained by Working Example 2, the thin portions was modified to the flattened portions. As a result, it became apparent that a risk of generating cracks was significantly reduced compared with the vessel base material obtained by Comparative Example 2.

### [Test 3]

In the test, an experiment that simultaneously used the four primary molds to form the uneven pattern structure on the vessel base material, and then used the secondary mold to modify the protruding portion and the thin portions formed on the vessel base material to the flattened portions was conducted.

The same one as the test 1 was used as the primary mold.

As illustrated in Fig. 5, a secondary mold that allowed modifying the protruding portion and the thin portions generated when the four primary molds were adjacently disposed to the flattened portions was manufactured. Specifically, using an aluminum material of 240 × 180 mm, it was manufactured by the method similar to the test 1.

A width of a boundary projecting portion in the secondary mold was configured to be 2 mm, and a width of the peripheral edge projecting portion 22 was configured to be 6 mm. Heights of the boundary projecting portion and the peripheral edge projecting portion were both 5 mm.

As the vessel base material, a linear low-density polyethylene film (manufactured by Toyo Seikan Group Holdings, Ltd.) having a thickness of 0.11 mm and 300 × 240 mm was used. The vessel base material was bonded to a reinforcing material made of polyethylene terephthalate using an acrylic-based adhesive material. At this time, the thickness of the reinforcing material was 0.06 mm, and a thickness of the sticking layer was 0.02 mm.

Next, as illustrated in Fig. 5(a), on an opposite surface of a bonding surface of the reinforcing material in the vessel base material, the four primary molds were adjacently disposed in the longitudinal and the short side direction and an uneven pattern structure was processed on a surface of the vessel base material using a thermal transfer apparatus (manufactured by Toyo Seikan Group Holdings, Ltd.). At this time, a temperature of the primary molds was set to be 130°C and pressurization was performed twice on the four primary molds at 4000 N for 20 seconds.

The surface of the vessel base material was set as a culture surface of the cell culture vessel, and a method for producing the cell culture vessel using only the primary molds was used as Comparative Example 3.

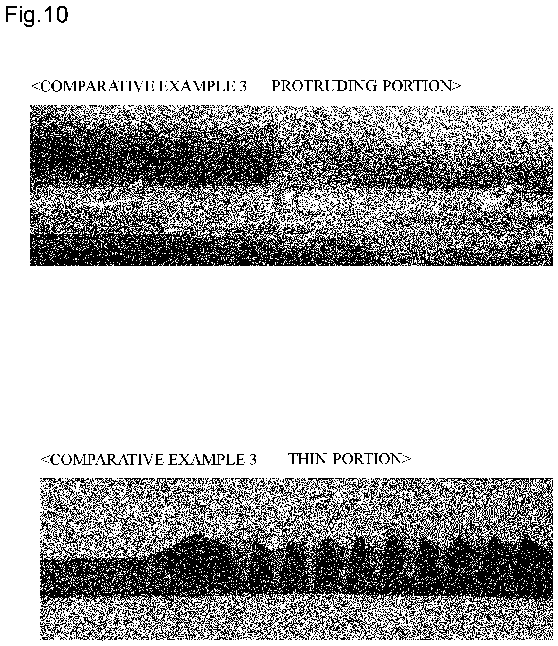

On the culture surface obtained by Comparative Example 3, a protruding portion was formed at a portion corresponding to a boundary line of the primary molds adjacent to one another. A thin portions was formed on a peripheral edge portion excluding the boundary line of the primary molds. These protruding portion and thin portions were photographed from side surfaces using a microscope to measure a height of the protruding portion from a bottom surface of the vessel base material and a thickness of the vessel base material in the thin portion. Fig. 10 illustrates the photographs of the protruding portion and the thin portion photographed at this time.

Next, the secondary mold was mounted on the thermal transfer apparatus and the secondary mold was pressed against the surface of the vessel base material on which the uneven pattern structure was processed. At this time, a temperature of the secondary mold was set to 130°C and pressurization was performed twice on the four primary molds at 4000 N for 20 seconds.

Accordingly, the secondary mold was pressed against the protruding portion formed at the portion corresponding to the boundary line of the primary mols adjacent to one another and the thin portions to modify the protruding portion and the thin portions to the flattened portions.

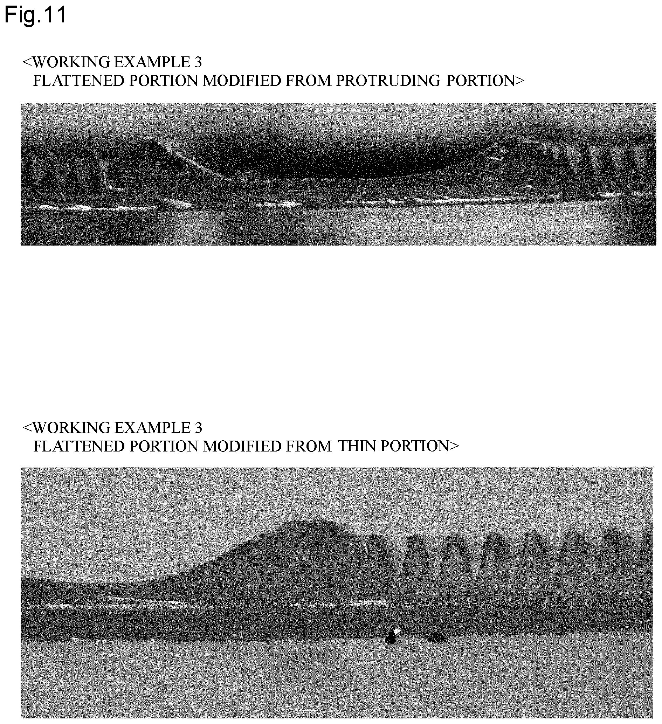

The surface of the vessel base material was set as a culture surface of the cell culture vessel, and a method for producing the cell culture vessel using the primary molds and the secondary mold was used as Working Example 3. The flattened portions were photographed from side surfaces using the microscope to measure a height of the flattened portion modified from the protruding portion from a bottom surface of the vessel base material and a thickness of the vessel base material in the flattened portion modified from the thin portion. Fig. 11 illustrates the photographs of the flattened portion modified from the protruding portion and the flattened portion modified from the thin portion photographed at this time.

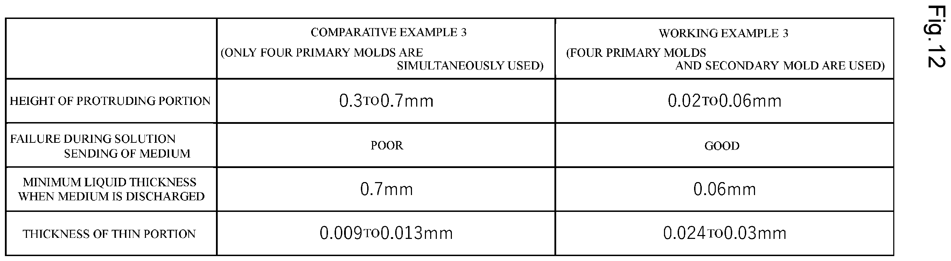

Fig. 12 shows results and effects of Comparative Example 3 and Working Example 3.

As shown in the diagram, the height of the protruding portions was from 0.3 to 0.7 mm, and a height at the highest portion of the protruding portion was 0.7 mm in Comparative Example 3. In contrast to this, the height of the flattened portions modified from the protruding portions was from 0.02 to 0.06 mm and the height of the highest portion of the flattened portion was 0.06 mm in Working Example 3.

Thus, it became apparent that while the protruding portion is present in the culture surface and therefore a failure possibly occurs during solution sending of the medium in Comparative Example 3, in Working Example 3, since the protruding portion is modified to the flattened portion, a risk of a failure during solution sending of the medium can be reduced.

It became apparent that since a minimum liquid thickness when the medium is discharged from the cell culture vessel was 0.7 mm in Comparative Example 3 but can be 0.06 mm in Working Example 3, the liquid thickness in the cell culture vessel can be decreased during medium exchange, and the amount of consumption of the medium can be reduced.

The thickness of the thin portions in Comparative Example 3 was from 0.009 to 0.013 mm, and a thickness of the thinnest portion of the thin portions was 0.009 mm. In contrast to this, the height of the flattened portions modified from the thin portions was from 0.024 to 0.03 mm, and the thickness of the thinnest portion of the flattened portion was 0.024 mm in Working Example 3.

That is, the vessel base material obtained by Working Example 3 had no thin portion, and a crack was less likely to occur compared with the one obtained by Comparative Example 3.

The present invention is not limited to the above-described embodiments and working examples, and, needless to say, various modifications can be made within the scope of the present invention. For example, the number of primary molds used to form one culture surface is, for example, larger than six, eight, or the like and the secondary mold is repeatedly pressed multiple times. Thus, appropriate change, such as modification of the protruding portion and the thin portions in the vessel base material formed by the primary molds to the flattened portions, can be made.

### INDUSTRIAL APPLICABILITY

The present invention can be preferably used to produce the cell culture vessel having the large-sized culture surface.

The entire contents of the documents described in the Description and Japanese Application as the basis of the priority claimed under the Paris Convention are hereby incorporated by reference.

### DESCRIPTION OF REFERENCE SIGNS

- 10: Primary mold
- 11: Projecting portion
- 12: Supporting portion
- 20, 20b: Secondary mold
- 21, 21b: Boundary projecting portion
- 22, 22b: Peripheral edge projecting portion
- 23, 23b: Supporting portion
- 30: Vessel base material
- 31: Uneven pattern structure
- 32: Protruding portion
- 33: Thin portion
- 34: Boundary flattened portion
- 35: Peripheral edge flattened portion
- 60: Reinforcing material

## Claims

1. A method for producing a cell culture vessel having a culture surface of cells, comprising:
using a primary mold including a projecting portion to form an uneven pattern structure on a surface of a vessel base material and a secondary mold including a projecting portion to flatten a part of the surface of the vessel base material;
pressing the primary mold against the vessel base material to form the uneven pattern structure on the vessel base material;
pressing the secondary mold against a thin portion formed on the vessel base material to modify the thin portion to a flattened portion; and
configuring the surface of the vessel base material including the uneven pattern structure formed with at least a part of the modified flattened portion as the culture surface to form the cell culture vessel.

2. The method for producing the cell culture vessel according to claim 1, comprising:
adjacently disposing a plurality of the primary molds along a boundary line and pressing the plurality of primary molds against the vessel base material to form the uneven pattern structure on the vessel base material; and
pressing the secondary mold against a protruding portion formed at a portion corresponding to the boundary line of the plurality of primary molds and the thin portion in the vessel base material to modify the protruding portion and the thin portion to the flattened portions.

3. The method for producing the cell culture vessel according to claim 1, comprising:
pressing the vessel base material multiple times using the one primary mold to form the uneven pattern structure on the vessel base material; and
pressing the secondary mold against the thin portion formed at a portion corresponding to a peripheral edge portion of the primary mold in the vessel base material to modify the thin portion to the flattened portion.

4. The method for producing the cell culture vessel according to any one of claims 1 to 3, comprising
pressing the thin portion and a predetermined region inside the thin portion by the secondary mold.

5. The method for producing the cell culture vessel according to any one of claims 1 to 4, comprising:
bonding the vessel base material to a reinforcing material; and
pressing the primary mold and the secondary mold against an opposite surface of a bonding surface of the reinforcing material in the vessel base material.

6. The method for producing the cell culture vessel according to any one of claims 1 to 5, wherein
the vessel base material is thermoplastic resin, and
the primary mold and the secondary mold are pressed to form the uneven pattern structure and the flattened portion on the vessel base material by thermal transfer or melt extrusion.

7. The method for producing the cell culture vessel according to any one of claims 1 to 6, comprising:
forming a plurality of approximately triangular prisms in parallel in a mountain range shape as the uneven pattern structure; or
forming a plurality of depressed portions with or without a fine hole.

8. The method for producing the cell culture vessel according to any one of claims 1 to 7, wherein
the cell culture vessel is a bag-shaped vessel internally having the culture surface of the cells.

9. A bag-shaped cell culture vessel internally having a culture surface of cells, comprising:
a plurality of regions on which an uneven pattern structure is formed on the culture surface;
a flattened portion disposed between a plurality of regions on which the uneven pattern structure is formed; and
a flattened portion at an entire peripheral edge of the culture surface.

10. The cell culture vessel according to claim 9, wherein
the uneven pattern structure is a plurality of approximately triangular prisms disposed in parallel in a mountain range shape or a plurality of depressed portions with or without a fine hole.
